(19) Europäisches Patentamt
European Patent Office
Office européen des brevets

(11) Publication number: 0 257 918
A1

(12) **EUROPEAN PATENT APPLICATION**

(21) Application number: 87307125.2

(51) Int. Cl.⁴: C07D 401/04 , A61K 31/47

(22) Date of filing: 12.08.87

(30) Priority: 15.08.86 GB 8619902

(43) Date of publication of application:
02.03.88 Bulletin 88/09

(84) Designated Contracting States:
AT BE CH DE ES FR GB GR IT LI LU NL SE

(71) Applicant: Pfizer Limited
Ramsgate Road
Sandwich Kent CT13 9NJ(GB)

(72) Inventor: Campbell, Simon Fraser
Grey Friars Upper Street
Kingsdown Deal Kent(GB)
Inventor: Roberts, David Anthony
Great Knott Giants Wood Lane
Congleton Cheshire(GB)
Inventor: Morris, David Stuart
26, Wellesley Road
Westgate-on-Sea Kent(GB)

(74) Representative: Wood, David John et al
Pfizer Limited Ramsgate Road
Sandwich Kent CT13 9NJ(GB)

(54) **Quinolone cardiac stimulants.**

(57) A quinolone cardiac stimulant of the formula:-

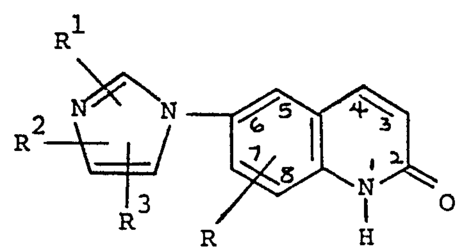

--- (I)

or a pharmaceutically acceptable salt thereof,
wherein R, which is attached to the 5-, 7-or 8-position of the quinolone, is H or $C_1$-$C_4$ alkyl;
$R^1$ is a group of the formula -$S(O)_n$.$C_1$-$C_4$ alkyl where n is 0, 1 or 2;
and $R^2$ and $R^3$, which can be the same or different, are H or $C_1$-$C_4$ alkyl.

EP 0 257 918 A1

# QUINOLONE CARDIAC STIMULANTS

## DESCRIPTION

This invention relates to imidazolyl-quinolone cardiac stimulants which in general selectively increase the force of myocardial contraction without producing significant increases in the heart rate. The compounds are useful in the curative or prophylactic treatment of cardiac conditions, in particular in the treatment of congestive heart failure.

Thus according to the invention there are provided substituted 2-(1H)-quinolones of the formula:-

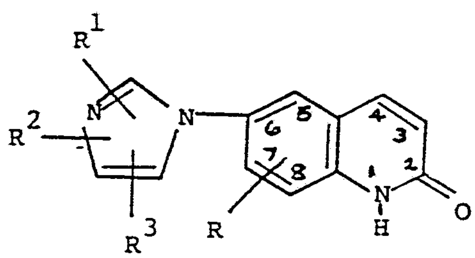

--- (I)

and their pharmaceutically acceptable salts,
wherein R, which is attached to the 5-, 7-or 8-position of the quinolone, is H or $C_1$-$C_4$ alkyl;
$R^1$ is a group of the formula $-S(O)_n.C_1$-$C_4$ alkyl where n is 0, 1 or 2;
and $R^2$ and $R^3$, which can be the same or different, are H or $C_1$-$C_4$ alkyl.

Although the compounds of the formula (I) are written as 2-(1H)-quinolones, it should be realized that the following tautomerism can occur:-

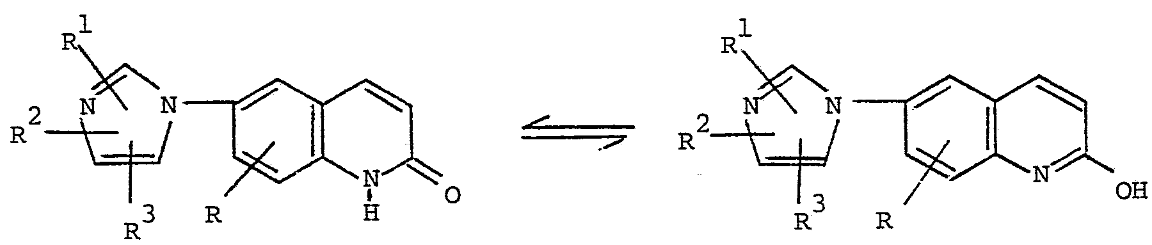

However, as the keto form is considered the more stable tautomer, the end products herein will be named and illustrated as quinolones although those skilled in the art will realise that both tautomers may be present or that any particular compound so named may exist predominantly as the hydroxy tautomer and the following disclosure is to be interpreted to incorporate all tautomeric forms.

The preferred alkyl groups are methyl. R is preferably a methyl group in the 8-position.

A preferred individual compound has the formula:-

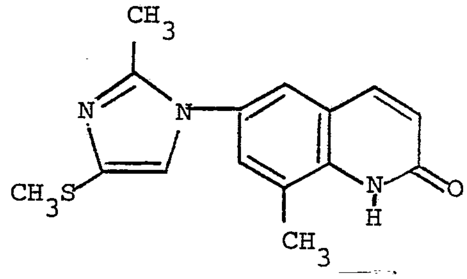

--- (IA)

The pharmaceutically acceptable salts of the compounds of the formula (I) are either acid addition salts formed from acids which form non-toxic acid addition salts containing pharmaceutically acceptable anions, such as hydrochloride, hydrobromide, hydroiodide, sulphate or bisulphate, phosphate or hydrogen phosphate, acetate, maleate, fumarate, lactate, tartrate, citrate, gluconate, methanesulphonate, and p-toluenesulphonate salts, or are metal salts, particularly the alkaline earth or alkali metal salts. The preferred metal salts are the sodium and potassium salts. All the salts are preparable by conventional techniques.

The cardiac stimulant activity of the compounds of the formula (I) is shown by their effectiveness in one or more of the following tests: (a) increasing the force of contraction in the "Starling" dog heart - lung preparation measured via a left ventricular catheter; (b) increasing myocardial contractility (left ventricular dp/dt max.) in the anaesthetised dog measured via a left ventricular catheter; (c) increasing myocardial contractility in the conscious dog with an implanted left ventricular transducer (dp/dt max.) or an exteriorised carotid artery loop (systolic time intervals).

In test (a), the positive inotropic effect of the test compound following bolus administration is measured in the "Starling" dog heart-lung preparation. The selectivity for increase in force versus frequency of contraction of the test compound is obtained.

In test (b), the positive inotropic action of the test compound following intravenous administration is measured in the anaesthetised dog. The magnitude and duration of this action, and the selectivity for increase in force versus frequency of contraction of the test compound are obtained, as are the peripheral effects, e.g. the effect on blood pressure.

In test (c) the positive inotropic action of the test compound following intravenous or oral administration to a conscious dog with an implanted left ventricular transducer (dp/dt max.) or an exteriorised cartoid artery loop (systolic time intervals) is measured. The magnitude of the inotropic action, the selectivity for increase in force versus frequency of contraction, and the duration of action of the inotropic effect of the test compound are all obtained.

The compounds of the formula (I) can be administered alone but will generally be administered in admixture with a pharmaceutical carrier selected with regard to the intended route of administration and standard pharmaceutical practice. For example they may be administered orally in the form of tablets containing such excipients as starch or lactose, or in capsules either alone or in admixture with excipients, or in the form of elixirs or suspensions containing flavouring or colouring agents. They may be injected parenterally, for example, intravenously, intramuscularly or subcutaneously. For parenteral administration, they are best used in the form of a sterile aqueous solution which may contain other solutes, for example, enough salts or glucose to make the solution isotonic.

For administration to man in the curative or prophylactic treatment of cardiac conditions such as congestive heart failure, it is expected that oral dosages of the compounds of the invention will be in the range from 1mg to 1g daily, taken in 1 to 4 divided doses per day, for an average adult patient (70kg). Dosages for intravenous administration would be expected to be within the range 0.5 to 100mg per single dose as required, for example in the treatment of acute heart failure. Thus for a typical adult patient, individual tablets or capsules might contain 1 to 250mg of active compound, in a suitable pharmaceutically acceptable vehicle or carrier. Variations may occur depending on the weight and condition of the subject being treated as will be known to medical practitioners.

Thus the present invention provides a pharmaceutical composition comprising a compound of the formula (I) as defined above or pharmaceutically acceptable salt thereof, together with a pharmaceutically acceptable diluent or carrier.

The invention also provides a method of stimulating the heart of a human being, which comprises administering to said human a compound of formula (I) or pharmaceutically acceptable salt thereof, or a pharmaceutical composition as defined above, in an amount sufficient to stimulate the heart of said human.

The invention yet further provides a compound of the formula (I) or pharmaceutically acceptable salt thereof, for use as a medicament, in particular for use in stimulating the heart of a human being suffering from congestive heart failure.

The invention also includes the use of a compound of the formula (I), or of a pharmaceutically acceptable salt thereof, for the manufacture of a medicament for stimulating the heart of a human being:-

The compounds of the formula (I) can be prepared by the following routes:-

Route A

This route prepares compounds in which the imidazolyl group has a $C_1$-$C_4$ alkylthio substituent (and, of course, optionally one or two $C_1$-$C_4$ alkyl substituents). It involves the reaction of the corresponding compounds having an iodo substituent on the imidazolyl group with a metal salt of a $C_1$-$C_4$ alkanethiol, preferably an alkali metal salt such as a sodium or potassium salt, e.g. sodium thiomethoxide.

The reaction can be illustrated in general terms as follows:-

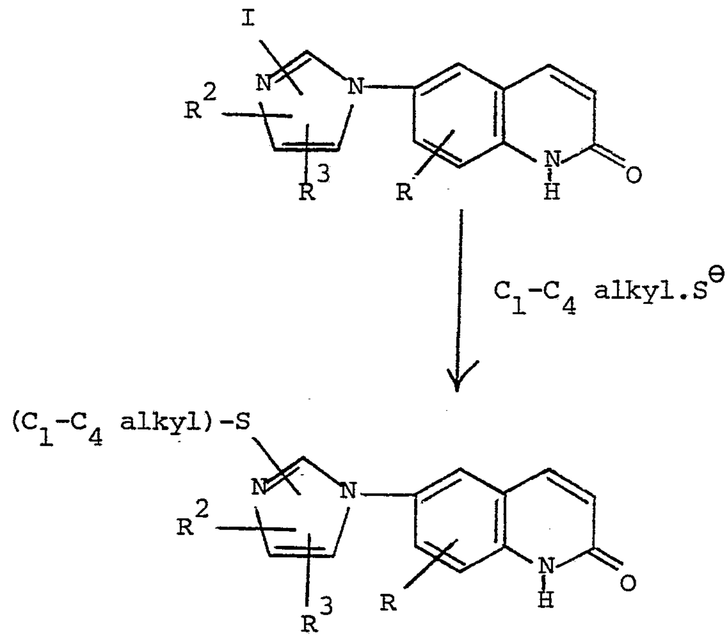


The reaction is preferably carried out by heating the starting iodo-substituted compound with the thioalkoxide in the presence of a copper (I) halide salt in a suitable organic solvent, e.g. 1-methyl-2-pyrrolidinone, at up to the reflux temperature, typically at 60-180°C, generally for 1-48 hours. The product can then be isolated and purified by conventional means.

A typical reaction is illustrated as follows:-

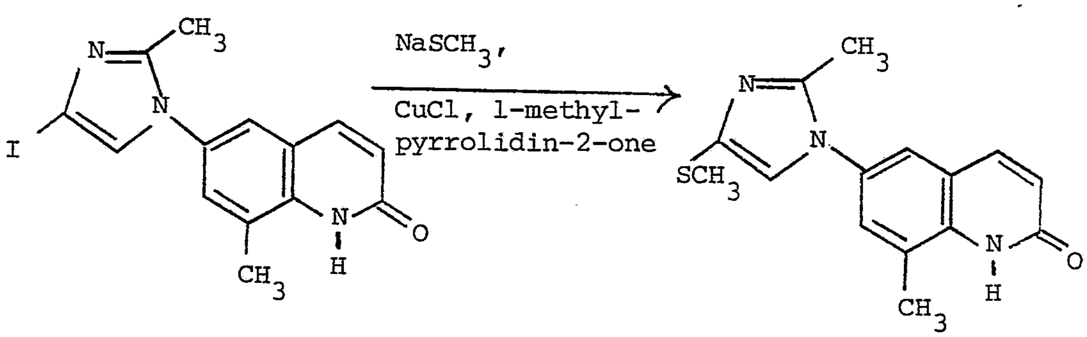


The iodo-containing starting materials can be prepared by conventional procedures, e.g. by the techniques described in European patent application publication no. 0166533 and in the additional information placed in the public file of that application in November 1985.

Route B

This route prepares compounds in which the imidazolyl group has a $C_1$-$C_4$ alkylsulphinyl substituent, and involves the reaction of the corresponding compound having a $C_1$-$C_4$ alkylthio substituent with a suitable oxidising agent, e.g. meta-chloroperbenzoic acid or hydrogen peroxide.

The reaction is typically carried out by stirring the alkylthio-substituted starting material with m eta-chloroperbenzoic acid (m-CPBA) in a suitable organic solvent, e.g. chloroform, at about 0°C for 0.5-10 hours. The product can then be isolated and purified by conventional means.

A typical reaction of this type is illustrated as follows:-

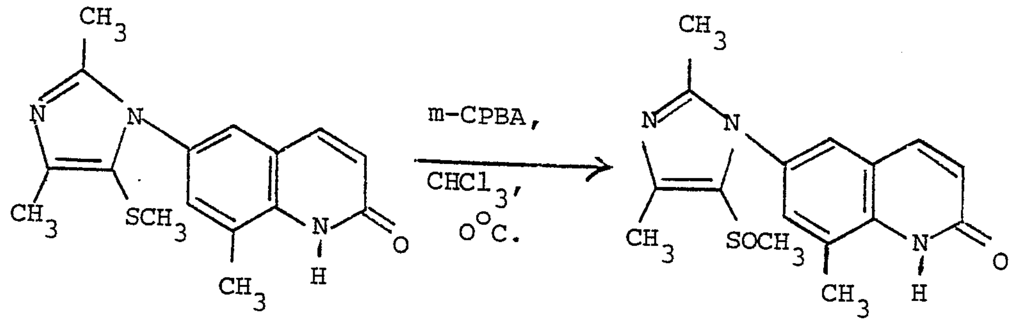


Route C

This route prepares compounds in which the imidazolyl group has a $C_1$-$C_4$ alkylsulphonyl substituent, and involves the reaction of the corresponding compound having a $C_1$-$C_4$ alkylsulphinyl substituent with a suitable oxidising agent.

The reaction is preferably carried out by stirring the alkylsulphinyl-substituted starting material with meta -chloroperbenzoic acid (m-CPBA) in a suitable organic solvent, e.g. chloroform, at up to 30°C (e.g. at room temperature) for 0.5-10 hours. The product can then be isolated and purified by conventional means.

A typical reaction of this type is illustrated as follows:-

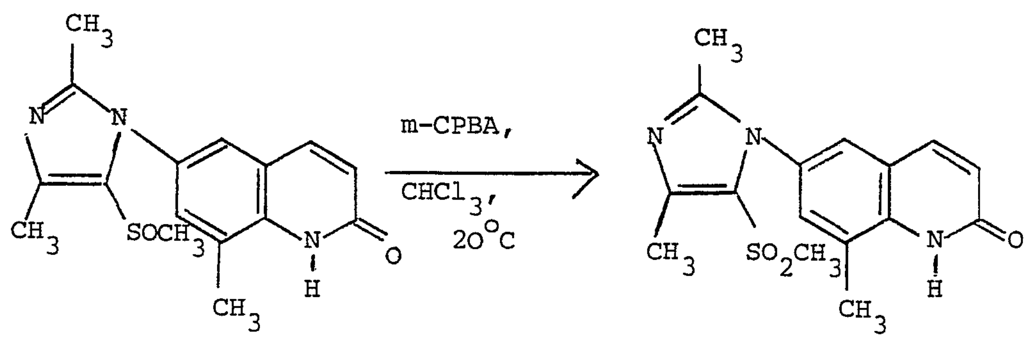


It is also possible to oxidise the $C_1$-$C_4$ alkylthio compounds directly to the $C_1$-$C_4$ alkylsulphonyl compounds using an excess (at least two equivalents) of oxidising agent.

Where the compounds of the invention contain one or more asymmetric centres, then the invention includes the separated enantiomers and diastereoisomers or mixtures thereof. The separated forms can be obtained by conventional means.

Salts can also be obtained conventionally.

The following Examples illustrate the invention (all temperatures are in °C):-

EXAMPLE 1

Preparation of 6-(2-methyl-4-methylthioimidazol-1-yl)-8-methyl-2-(1H)-quinolone, 0.5 $H_2O$

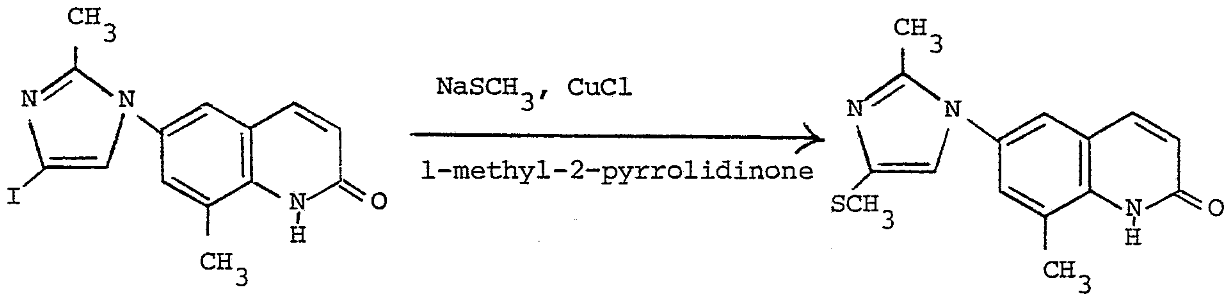


6-(4-Iodo-2-methlimidazol-1-yl)-8-methyl-2-(1H)-quinolone (1.4 g) was added to a stirred suspension of sodium thiomethoxide (0.8 g) and copper (I) chloride (0.45 g) in 1-methyl-2-pyrrolidinone (10 $cm^3$) and the mixture heated at 140°C under nitrogen. After 6 hours the cooled mixture was poured into aqueous ammonia solution (10 $cm^3$, S.G. 0.880) and extracted with methanol:dichloromethane, 1:20 by volume (3 x 75 $cm^3$). The combined and dried ($MgSO_4$) organic extracts were filtered and evaporated in vacuo and the residue was chromatographed on silica (Merck "MK 60.9385" [Trade Mark]) eluting with methanol:dichloromethane, 1:20 by volume.

Combination and evaporation of the appropriate fractions afforded a solid which was recrystallised from ethyl acetate to give the title compound, m.p. 255°, (0,48 g).

Analysis %:-

Found: C,60.8; H,5.3; N,14.3;
Calculated for $C_{15}H_{15}N_3OS$, 0.5 $H_2O$: C,61.1; H,5.5; N,14.3.

EXAMPLE 2

Preparation of 6-(2,4-dimethyl-5-methylthioimidazol-1-yl)-8-methyl-2-(1H)-quinolone, 0.25 $H_2O$

The title compound, m.p. 263-5°, was prepared similarly to the procedure of Example 1 using 6-(2,4-dimethyl-5-iodoimidazol-1-yl)-8-methyl-2-(1H)-quinolone (see Example 40 of European patent application publication no. 0166533), sodium thiomethoxide and copper (I) chloride as the starting materials.

Analysis %:-

Found: C,63.3; H,5.7; N,13.9;
Calculated for $C_{16}H_{17}N_3OS$, 0.25 $H_2O$: C,63.3; H,5.8; N,13.8.

EXAMPLE 3

Preparation of 6-(2,4-dimethyl-5-methylsulphinylimidazol-1-yl)-8-methyl-2-(1H)-quinolone, 0.33 $H_2O$

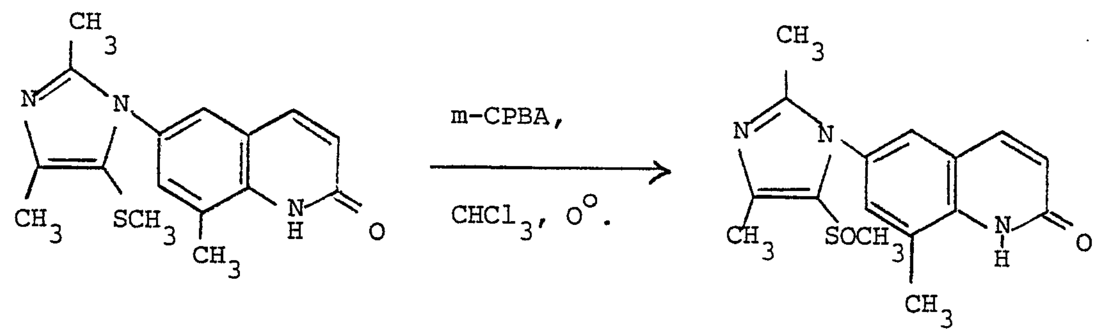


meta-Chloroperbenzoic acid (0.3 g) ("m-CPBA") was added in portions to a stirred solution of 6-(2,4-dimethyl-5-methylthioimidazol-1-yl)-8-methyl-2-(1H)-quinolone (10.52 g) in chloroform (20 cm³) at 0°. After 30 minutes the solution was basified with saturated sodium carbonate solution to pH 10 (approximately) and extracted with dichloromethane (3 x 50 cm³). The combined and dried ($MgSO_4$) organic extracts were filtered and evaporated in vacuo and the residue was chromatographed on silica (Merck "MK 60.9385" [Trade Mark]) eluting with methanol:dichloromethane, 1:20 by volume. Combination and evaporation of appropriate fractions afforded a solid which was recrystallised from ethyl acetate to give the title compound, m.p. 283-5°C, (0.32 g).

Analysis %:-

Found: C,59.9; H,5.5; N,13.0;
Calculated for $C_{16}H_{17}N_3O_2S$, 0.33 $H_2O$: C,59.8; H,5.5; N,13.1.

## EXAMPLE 4

Preparation of 6-(2-methyl-4-methylsulphinylimidazol-1-yl)-8-methyl-2-(1H)-quinolone, 1.25 $H_2O$

This compound, m.p. 292°, was prepared similarly to the procedure of Example 3 using 6-(2-methyl-4-methylthioimidazol-1-yl)-8-methyl-2-(1H)-quinolone and meta-chloroperbenzoic acid as the starting materials.

Analysis %:-

Found: C,55.6; H,4.9; N,12.7;
Calculated for $C_{15}H_{15}N_3O_2S$, 1.25 $H_2O$: C,55.6; H,5.4; N,13.0.

## EXAMPLE 5

Preparation of 6-(2,4-dimethyl-5-methylsulphonyl-imidazol-1-yl)-8-methyl-2-(1H)-quinolone

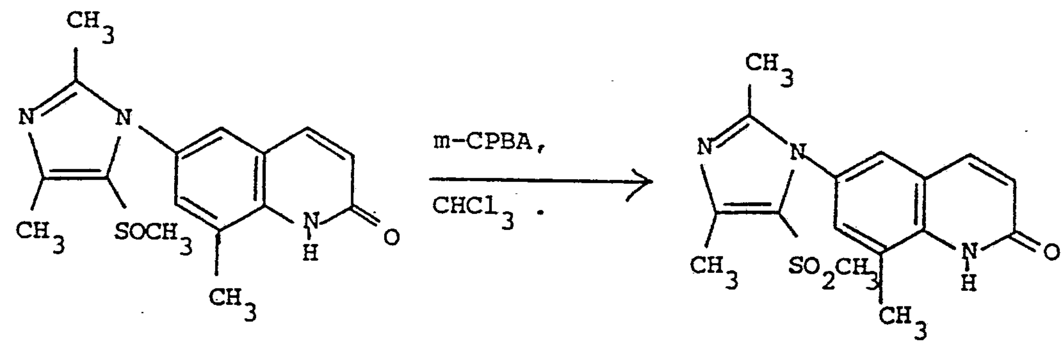


meta-Chloroperbenzoic acid (0.11 g) was added to a stirred solution of 6-(2,4-dimethyl-5-methylsulphinylimidazol-1-yl)-8-methyl-2-(1H)-quinolone (0.15 g) in chloroform (10 $cm^3$) at room temperature. After 5 hours the solution was basified with saturated sodium carbonate solution to pH 10 (approximately) and extracted with chloroform (3 x 50 $cm^3$). The combined and dried ($MgSO_4$) organic extracts were filtered and evaporated in vacuo and the residue was chromatographed on silica (Merck "MK 60.9385" [Trade Mark]) eluting with methanol:dichloromethane, 1:20 by volume. Combination and evaporation of appropriate fractions afforded a solid which was recrystallised from ethyl acetate to give the title compound, m.p. 276-8°C, (0.06 g).

Analysis %:-

Found: C,58.3; H,5.4; N,12.3;
Calculated for $C_{16}H_{17}N_3O_3S$: C,58.0; H,5.1; N,12.7.

## EXAMPLE 6

Preparation of 6-(2-methyl-4-methylsulphonylimidazol-1-yl)-8-methyl-2-(1H)-quinolone

This compound, m.p. 332-3°, was prepared similarly to the procedure of Example 5 using 6-(2-methyl-4-methylsulphinylimidazol-1-yl)-8-methyl-2-(1H)-quinolone and meta-chloroperbenzoic acid as the starting materials.

Analysis %:-

Found: C,56.4; H,4.7; N,12.9;
Calculated for $C_{15}H_{15}N_3O_3S$: C,56.8; H,4.8; N,13.2.

The following Preparations describe the preparation of certain of the starting materials used in the previous Examples.

Preparation 1

6-(4-Iodo-2-methylimidazol-1-yl)-8-methyl-2-(1H)-quinolone, 1/3 $H_2O$, m.p. 285-287°, was prepared similarly to Example 12 of European patent application publication no. 0166533 starting from trans-1-[4-{N-(3-ethoxypropenamido)}-3-methyl-phenyl]-4-iodo-2-methylimidazole and 98% w/w sulphuric acid.

Analysis %:-

Found: C,45.3; H,3.3; N,11.3;
Calculated for $C_{14}H_{12}N_3OI.1/3\ H_2O$: C,45.3; H,3.4; N,11.3.

Preparation 2

Trans-1-[4-{N-(3-ethoxypropenamido)} -3-methylphenyl]-4-iodo--2-methylimidazol, m.p. 172-174°, was prepared similarly to Preparation 1 of European patent application publication no. 0166533 using 1-(4-amino-4-methylphenyl)-4-iodo-2-methylimidazole and trans-3-ethoxypropenoyl chloride as the starting materials with anhydrous pyridine as the solvent.

Analysis %:-

Found: C,46.8; H,4.5; N,10.1;
Calculated for $C_{16}H_{18}N_3O_2I$: C,46.7; H,4.4; N,10.2.

Preparation 3

1-(4-Amino-3-methylphenyl)-4-iodo-2-methylimidazole, isolated and later used as a crude oil, was prepared similarly to Preparation 12 of the said European patent application using 1-(3-methyl-4-nitrophenyl)-4-iodo-2-methylimidazole and stannous chloride dihydrate as the starting materials with absolute ethanol as the solvent.

Preparation 4

1-(3-Methyl-4-nitrophenyl)-4-iodo-2-methylimidazole, m.p. 146-148°, was prepared similarly to Preparation 20 of the said European patent application using 4-fluoro-2-methylnitrobenzene, 4-iodo-2-methylimidazole and sodium carbonate as the starting materials with dimethylformamide as the solvent.

Analysis %:-

Found: C,38.5; H,3.1; N,12.4;
Calculated for $C_{11}H_{10}N_3O_2I$: C,38.5; H,2.9; N,12.2.

Preparation 5

4-Iodo-2-methylimidazole

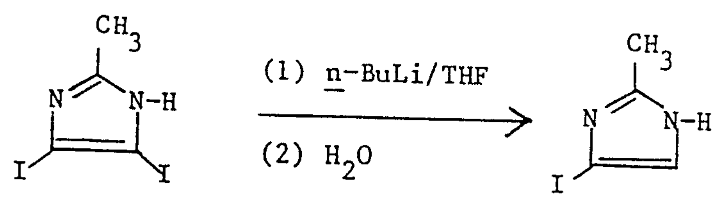


n-Butyllithium (86 cm$^3$ of a 1.43 M solution is n-hexane) was added dropwise to a stirred solution of 4,5-diiodo-2-methyl-imidazole (20.5 g) in tetrahydrofuran (THF) (300 cm$^3$) at -70° under nitrogen. After 15 minutes water (20 cm$^3$) was added and the mixture was warmed to room temperature over 1 hour. The mixture was then evaporated in vacuo to low bulk, more water (50 cm$^3$) was added, and the pH was adjusted to 8 by addition of 2M hydrochloric acid. The aqueous phase was extracted with dichloromethane (3 x 150 cm$^3$), and the combined and dried ($MgSO_4$) organic extracts were evaporated in vacuo to give a residue which was chromatographed on silica (Merck "MK 60.9385" [Trade Mark]). Elution with ethyl acetate followed by combination and evaporation of appropriate fractions, gave 4-iodo-2-methylimidazole as a solid (9.0 g) which was characterised spectroscopically and used directly without further purification.

Preparation 6

4,5-Diido-2-methylimidazole

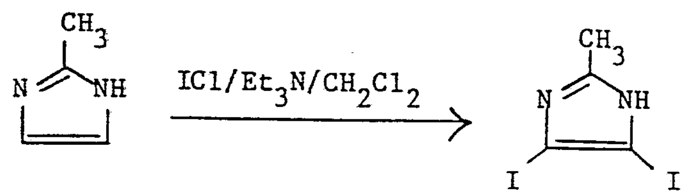


A solution of iodine monochloride (32.5 g) dissolved in dichloromethane (100 $cm^3$) was added dropwise over 1.5 hours to a solution of 2-methylimidazole (8.2 g) and triethylamine (20.2 g) in dichloromethane (200 $cm^3$) at -70° under nitrogen. The mixture was stirred for a further 30 minutes, warmed to -30°, and then poured into water (200 $cm^3$). The resulting precipitate was filtered off, dried and recrystallised from ethyl acetate-hexane to afford 4,5-diiodo-2-methylimidazole (18.5 g) which was characterised spectroscopically and used directly without further purification.

**Claims**

1. A quinolone of the formula:-

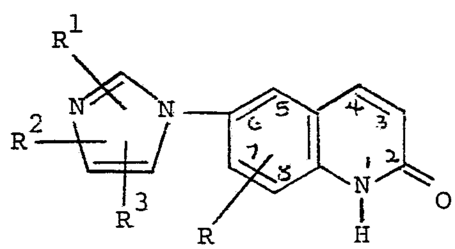

--- (I)

or a pharmaceutically acceptable salt thereof,
wherein R, which is attached to the 5-, 7-or 8-position of the quinolone, is H or $C_1$-$C_4$ alkyl;
$R^1$ is a group of the formula -$S(O)_n$.$C_1$-$C_4$ alkyl where n is 0, 1 or 2;
and $R^2$ and $R^3$, which can be the same or different, are H or $C_1$-$C_4$ alkyl.

2. A compound as claimed in claim 1, wherein R is a methyl group in the 8-position.

3. 6-(2-Methyl-4-methylthioimidazol-1-yl)-8-methyl-2-(1H)-quinolone.

4. A pharmaceutical composition comprising a compound of the formula (I) as claimed in any one of the preceding claims, or a pharmaceutically acceptable salt thereof, and a pharmaceutically acceptable diluent or carrier.

5. A compound of the formula (I) as claimed in any one of claims 1 to 3, or a pharmaceutically acceptable salt thereof, for use as a medicament.

6. The use of a compound of the formula (I) as claimed in any one of claims 1 to 3, or of a pharmaceutically acceptable salt thereof, for the manufacture of a medicament for use as a cardiac stimulant.

Claims for the following contracting states: AT, ES and GR

1. A process for preparing a quinolone of the formula:-

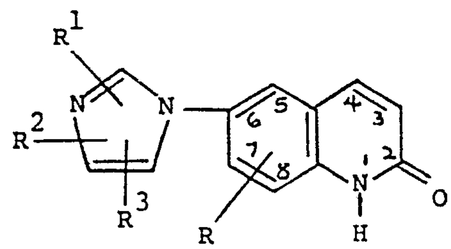

--- (I)

or a pharmaceutically acceptable salt thereof, wherein R, which is attached to the 5-, 7-or 8-position of the quinolone, is H or $C_1$-$C_4$ alkyl; $R^1$ is a group of the formula $-S(O)_n.C_1$-$C_4$ alkyl where n is 0, 1 or 2; and $R^2$ and $R^3$, which can be the same or different, are H or $C_1$-$C_4$ alkyl; characterised by reacting a compound of the formula:-

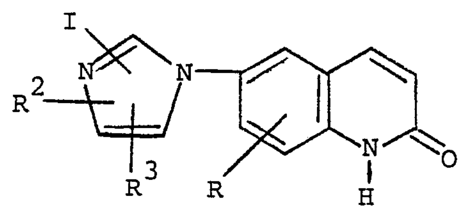

wherein R, $R^2$ and $R^3$ are as defined above, with a metal salt of a $C_1$-$C_4$ alkanethiol so as to produce a compound of the formula (I) in which $R^1$ is $-S.(C_1$-$C_4$ alkyl), said process being followed by, optionally, one or more of the following steps:-

(a) oxidation of a compound of the formula (I) in which n is zero to a compound of the formula (I) in which n is 1 or 2;

(b) oxidation of a compound of the formula (I) in which n is 1 to a compound of the formula (I) in which n is 2; and

(c) conversion of a compound of the formula (I) into a pharmaceutically acceptable salt.

2. A process according to claim 1, characterised in that the metal salt of the alkanethiol is the sodium or potassium salt, and in that the reaction is carried out in the presence of a copper (I) halide.

3. A process according to claim 2, characterised in that the copper halide is copper (I) chloride.

4. A process according to any one of the preceding claims, characterised in that it is used to prepare a compound in which R is a methyl group in the 8-position.

5. A process according to claim 4, characterised in that 6-(2-methyl-4-methylthioimidazol-1-yl)-8-methyl-2-1(H)-quinolone is prepared by reacting 6-(4-iodo-2-methylimidazol-1-yl)-8-methyl-2-(1H)-quinolone with sodium thiomethoxide in the presence of copper (I) chloride.

6. A process for preparing a pharmaceutical composition, characterised by mixing a compound of the formula (I) as defined in claim 1, or a pharmaceutically acceptable salt thereof, with a pharmaceutically acceptable diluent or carrier.

European Patent Office

# EUROPEAN SEARCH REPORT

Application number

EP 87 30 7125

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | EP-A-0 166 533 (PFIZER LTD.)<br>* claims 1-10; examples 1-7, 10, 12, 13, 20-23, 33-36, 39-42 *<br>--- | 1,2,4-6 | C 07 D 401/04<br>A 61 K 31/47 |
| A | EP-A-0 148 623 (PFIZER LTD.)<br>* claims 1, 20, 21 *<br>--- | 1,4-6 | |
| A | EP-A-0 052 016 (OTSUKA PHARMACEUTICAL CO. LTD.)<br>* claims 1, 18 *<br>----- | 1,4-6 | |

TECHNICAL FIELDS SEARCHED (Int. Cl.4)

C 07 D 401/00
A 61 K 31/00

The present search report has been drawn up for all claims

| Place of search | Date of completion of the search | Examiner |
|---|---|---|
| BERLIN | 28-10-1987 | VAN AMSTERDAM L.J.P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document